# EUROPÄISCHE PATENTSCHRIFT

(11) **EP 3 049 385 B1**
(45) Veröffentlichungstag und Bekanntmachung des Hinweises auf die Patenterteilung: **03.01.2018**
(21) Anmeldenummer: 14757910.6
(22) Anmeldetag: 27.08.2014
(51) Int. Cl.: C07C 67/39, C07C 45/75, C07C 45/84, C07C 47/22, C07C 69/54

(54) **VERFAHREN ZUR HERSTELLUNG VON METHYLMETHACRYLAT**
PROCESS FOR PRODUCING METHYL METHACRYLATE
PROCÉDÉ DE FABRICATION DE MÉTHACRYLATE DE MÉTHYLE

(30) Priorität: 26.09.2013 EP 13186137
(43) Veröffentlichungstag der Anmeldung: 03.08.2016
(73) Patentinhaber: Evonik Röhm GmbH, 64293 Darmstadt (DE)
(72) Erfinder: KRILL, Steffen, 64367 Mühltal (DE); GRÖMPING, Matthias, 64295 Darmstadt (DE); LYGIN, Alexander, 64347 Griesheim (DE); BALDUF, Torsten, 64319 Pfungstadt (DE); BURGHARDT, Rudolf, 64287 Darmstadt (DE)
(86) Internationale Anmeldenummer: PCT/EP2014/068171
(87) Internationale Veröffentlichungsnummer: WO 2015/043861

(56) Entgegenhaltungen:
- WO-A2-2012/154450
- CN-A- 1 817 844
- CN-A- 101 074 192
- US-A- 4 496 770
- US-A- 4 638 085
- US-A- 5 969 178

## Beschreibung

### Gebiet der Erfindung

Die vorliegende Erfindung betrifft ein Verfahren zur Herstellung von Methylmethacrylat durch direkte oxidative Veresterung von Methacrolein und die Herstellung von Methacrolein. Dabei zeichnet sich dieses neuartige Verfahren dadurch aus, dass die Ausbeute und die Effizienz des Prozesses gegenüber dem Stand der Technik durch eine Abfolge verschiedener Destillationsschritte deutlich gesteigert werden konnte.

### Stand der Technik

Methylmethacrylat (MMA) wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierender Spezialester, die z.B. durch Veresterung von MAS mit den entsprechenden Alkoholen hergestellt werden.

MMA wird heute überwiegend ausgehend von Blausäure und Aceton über das entstehende Acetoncyanhydrin (ACH) als Zentralintermediat hergestellt. Dieses Verfahren hat den Nachteil, dass sehr große Mengen an Ammoniumsulfat erhalten werden, deren Aufbereitung mit sehr hohen Kosten verbunden ist. Weitere Verfahren, die eine andere Rohstoffbasis als ACH verwenden, sind in der einschlägigen Patentliteratur beschrieben und mittlerweile im Produktionsmaßstab realisiert worden. In diesem Zusammenhang werden heute auch C4 basierte Rohstoffe wie Isobutylen oder tert-Butanol (TBA) als Edukte, die über mehrere Verfahrensstufen in die gewünschten Methacrylsäurederivate umgewandelt werden, verwendet.

Hierbei wird im Allgemeinen Isobutylen oder tert-Butanol zu Methacrolein in einer ersten Stufe oxidiert, welches anschließend zu Methacrylsäure mit Sauerstoff umgesetzt wird. Die erhaltene Methacrylsäure wird nachfolgend mit Methanol in MMA überführt. Nähere Einzelheiten zu diesem Verfahren sind unter anderem in Ullmann's Encyclopedia of Industrial Chemistry 2012, Wiley-VCH Verlag GmbH & Co. KGaA, Weinheim, Methacrylic Acid and Derivatives, DOI: 10.1002/14356007.a16_441.pub2 und in "Trends and Future of Monomer-MMA Technologies", SUMITOMO KAGAKU 2004- II dargelegt.

Gemäß einer Abwandlung dieser Herstellungsmethode kann anstatt von einem C4-Baustein, wie Isobutylen, auch von Ethylen ausgegangen werden, welches mit Synthesegas zunächst zu Propanal und anschließend mit Formaldehyd zu Methacrolein umgesetzt wird. Das erhaltene Methacrolein wird mit Luft in der Gasphase am heterogenen Kontakt zu Methacrylsäure oxidiert und mit Methanol zu MMA verestert (Ullmann's Encyclopedia of Industrial Chemistry 2012, Wiley-VCH Verlag GmbH & Co. KGaA, Weinheim, Methacrylic Acid and Derivatives, DOI: 10.1002/14356007.al16_441.pub2 "Methacrylic Acid from Ethylene").

In einem weiteren Verfahren wird MMA durch Oxidation von Isobutylen oder TBA mit Luftsauerstoff in der Gasphase am heterogenen Katalysator zu Methacrolein und anschließender oxidativer Veresterungsreaktion von Methacrolein unter Verwendung von Methanol erhalten. Dieses von ASAHI entwickelte Verfahren ist unter anderem in den Druckschriften US 5,969,178 und US 7,012,039 beschrieben.

Problematisch an allen oben dargestellten Verfahren sind insbesondere weiterhin auch die relativ unbefriedigenden Ausbeuten, hohe Verluste bei den Oxidationsschritten und damit allgemein eine einhergehende Nebenproduktbildung - wie z.B. von CO₂, - die aufwendige Verfahrensschritte zur Isolierung des Produkts bedingen. So erreichen alle Verfahren, die von Isobutylen oder äquivalenten C4 basierten Rohstoffen wie TBA oder Methyl-tert-butylether (MTBE) ausgehen, in der Gasphasenoxidation an einem heterogenen Katalysatorsystem Ausbeuten unterhalb 90 %. In der einschlägigen Literatur sind Ausbeuten von unterhalb 85 % für die Methacroleinherstellung ausgehend von Isobutylen beschrieben (z.B. Tabelle 5 in Ullmann's Encyclopedia, siehe oben).

Darüber hinaus wird in der Patentanmeldung CN 101074192 ein Verfahren zur Herstellung von MMA beschrieben, bei dem zunächst aus Propanal und Formaldehyd bei einer Temperatur im Bereich von 40 bis 45 °C und einer Reaktionszeit im Bereich von 30 bis 100 Minuten Methacrolein gebildet wird, welches anschließend mit Methanol zu MMA oxidiert wird. Ein ähnliches Verfahren wird weiterhin von Yuchao Li et al. "Synthesis of methacrolein by condensation of propionaldehyde with formaldehyde", Advance Materials Research Vols. 396-398 (2012) pp 1094 - 1097 vorgeschlagen. Von einem Arbeiten bei erhöhter Temperatur oder einem Überdruck wird in dieser Druckschrift ausdrücklich abgeraten. Nachteilig an diesem Verfahren ist der hohe Bedarf an Säure und Amin, welche zur Katalyse der Reaktion eingesetzt werden. Hierdurch entstehen große Mengen an Abfallprodukten, da das Amin unter den genannten Bedingungen zu einem beachtlichen Anteil zerstört wird. Eine der Nebenreaktion, die den Katalysator desaktiviert, ist die Eschweiler-Clarke Reaktion, die zur Bildung von methyliertem tertiären Amin führt, das nicht mehr in der Lage ist, die Mannich Reaktion zu katalysieren (US 4,408,079, Spalte 2, Zeilen 15ff). So wird beispielsweise aus Dimethylamin Trimethylamin. Zur Lösung dieser Probleme wird in der europäischen Patentanmeldung mit dem Anmeldeaktenzeichen 13002076.1 vorgeschlagen, die Mannich-Reaktion der ersten Stufe bei höheren Drücken durchzuführen und für die zweite Stufe einen edelmetallhaltigen Katalysator zu verwenden.

Ein weiterer sehr großer Nachteil der direkten Kombination einer Mannich-Reaktion zur Methacrolein-Synthese in einer ersten Stufe und die anschließende oxidative Veresterung mit Methanol in einer zweiten Stufe ist jedoch, dass sich in der zweiten Stufe, bedingt durch einen erhöhten Wassergehalt des Roh-Methacroleins, vermehrt Methacrylsäure als Nebenprodukt bildet. Diese Methacrylsäure muss mit einer höheren Menge eines basischen Hilfsmittels, wie z.B. NaOH teilweise oder vollständig neutralisiert werden, damit das Zielprodukt MMA mit hohen Selektivitäten hergestellt werden kann und der eingesetzte Katalysator hohe Standzeit aufweist. Dadurch erhöhen sich die entstehende Abfallmenge und der Aufwand für deren Entsorgung. Die Rückgewinnung freier MAS oder wertvoller Folgeprodukten (MMA) aus dem so neutralisiertem Gemisch erfordert wiederum den Einsatz einer Säure und führt zu vermehrter Abfallproduktbildung. Diese Methacrylsäure muss nicht nur aufwendig entfernt werden und wirkt somit ausbeutesenkend, sondern ist auch schädlich für den in der zweiten Stufe verwendeten Katalysator. Dadurch kommt es zu häufigeren Stillständen und einem insgesamt höheren Einsatzstoffverbrauch. In JP 2003-192632 wird der Zusammenhang des Wassergehalts und einer Bildung von Methacrylsäure beschrieben. Es wird jedoch nur darauf hingewiesen, dass der Wassergehalt des Methacroleins vor der Verwendung in der oxidativen Veresterung reduziert werden sollte.

Für das weniger bevorzugte Verfahren des Standes der Technik, bei dem in der ersten Stufe gemäß den Druckschriften US 5,969,178 und US 7,012,039 Isobutylen oder tert-Butanol in der Gasphase zu Methacrolein umgesetzt werden, bestehen gleichfalls die Probleme bezüglich Wassergehaltes und der Methacrylsäurebildung. Daher lehren die US 5,969,178 und die US 7,012,039, dass das Methacrolein der ersten Stufe sehr aufwendig, mehrstufig gereinigt werden muss und insbesondere aufwendig entwässert werden muss, bevor das Produkt in die Anlage zur oxidativen Veresterung überführt werden kann.

Ein weiterer Nachteil der direkten Kombination einer Mannich-Reaktion und einer oxidativer Veresterung mit Methanol sind die Rohstoffe und Nebenprodukte, die aus der ersten Stufe in die oxidative Veresterung der zweiten Stufe übertragen werden und dort negative Wirkung auf die Raum-Zeit-Ausbeute und/oder die Katalysatorstandzeit und/oder den Methanolverbrauch haben. Bei diesen Rohstoffen bzw. Nebenprodukten handelt es sich neben dem bereits diskutierten Wasser insbesondere um dimeres Methacrolein sowie um Formaldehyd und dessen Folgeprodukte, insbesondere Oligo- und Polymere.

Direkte oxidative Veresterung vom dimeren Methacrolein mit Methanol steht bei begrenzter Kapazität des Katalysators in Konkurrenz zur gleichen Reaktion des monomeren Methacroleins und senkt damit die Raum-Zeit-Ausbeute der Hauptreaktion. Dies hat insbesondere eine große Bedeutung, da das dimere Methacrolein gegenüber dem monomeren Methacrolein in der oxidativen Veresterung bevorzugt umgesetzt wird.

Das bei der Herstellung von Methacrolein über eine Mannich Kondensation als Edukt eingesetzte Formaldehyd und dessen Folgeprodukte (insbesondere Oligo- und Polymere) führen gleich zu mehreren Problemen bei der direkten oxidativen Veresterung von Methacrolein. Formaldehyd selbst wirkt reduktiv, was zu einer Schädigung des in der oxidativen Veresterung eingesetzten Katalysators und damit zu verkürzten Katalysatorstandzeiten führen kann. Weiterhin kann sich unter den oxidativen Bedingungen Ameisensäure bilden, die ebenfalls schädlich für den Katalysator ist. Formaldehyd bildet bekanntlich reversibel eine Reihe von Oligo- und Polymeren (z.B. Paraformaldehyd). Die Hydroxyendgruppen solcher Oligo- und Polymeren aus Formaldehyd werden im Laufe der oxidativen Veresterung mit Methacrolein umgesetzt, was zu nun stabilen und unlöslichen Polymeren führen kann. Dies äußert sich in einer Trübung der Reaktionslösung und man beobachtet Feststoffablagerungen am Katalysatorbett, was wiederum zu Verstopfungen der Apparate und Verringerung der Katalysatorstandzeit führen kann.

WO 2012/154450 A2 schlägt zur Herstellung von Methylmethacrylat (MMA) vor, die Herstellung von Methacrolein aus Propanal und Formaldehyd gemäß US 4,496,770 mit einer direkten oxidativen Veresterung von Methacrolein mit Methanol zu MMA zu kombinieren. US 4,496,770 beschreibt die Herstellung von Methacrolein aus Propanal und Formaldehyd in Gegenwart von Aminsalzen sowie die Isolierung von flüssigem Roh-Methacrolein durch Destillation und anschließende Trennung von einer wässrigen Phase.

### Aufgaben

In Anbetracht des Standes der Technik ist es daher Aufgabe der vorliegenden Erfindung, ein technisch verbessertes Verfahren zur Herstellung von MMA zur Verfügung zu stellen, das nicht mit den Nachteilen herkömmlicher Verfahren behaftet ist.

Insbesondere soll es ermöglicht werden, MMA in einem mehrstufigen Prozess aus Propanal und Formaldehyd herzustellen, wobei gegenüber dem Stand der Technik die Ausbeute und die Anlagenbelegzeit gesteigert werden.

Insbesondere soll dazu die Bildung von Methacrylsäure während der direkten oxidativen Veresterung von Methacrolein mit Methanol zu MMA und damit der Verbrauch der Hilfsbase zur Einstellung des pH-Werts vermindert werden.

Insbesondere war es Aufgabe der vorliegenden Erfindung, den Verbrauch an Methanol durch die oxidative Veresterung von Nebenprodukten der Methacrolein-Synthese, wie z.B. dimeres Methacrolein, zu reduzieren.

Außerdem war es Aufgabe der vorliegenden Erfindung die negativen Auswirkungen von Formaldehyd und dessen Folgeprodukten auf die direkte oxidative Veresterung von Methacrolein zu reduzieren.

Ferner sollte die Verbesserung des Verfahrens durch eine sehr einfache und kostengünstige Modifikation bestehender Anlagen des Standes der Technik realisierbar sein und im Betrieb dieser Anlagen nur geringe Unterhaltskosten verursachen.

Darüber hinaus lag der vorliegenden Erfindung die Aufgabe zugrunde, dass das mit dem Verfahren hergestellte MMA und ein daraus hergestelltes Polymethylmethacrylat (PMMA) eine besonders geringe Färbung aufweist.

Weitere nicht explizit genannte Aufgaben ergeben sich aus dem Gesamtzusammenhang der nachfolgenden Beschreibung und der Ansprüche.

### Lösung

Gelöst werden diese Aufgaben durch ein neuartiges Verfahren zur Herstellung von MMA, das folgende Schritte umfasst:
A) Herstellung von Methacrolein aus Propanal und Formaldehyd in Gegenwart von Aminsalzen,
B) Isolierung von Roh-Methacrolein durch Destillation in einer ersten Destillationskolonne und anschließende Trennung von einer wässrigen Phase,
C) Destillation des aus Schritt B) gewonnenen Roh-Methacroleins in einer zweiten Destillationskolonne in Gegenwart von Methanol,
D) anschließende oxidative Veresterung des aus Schritt C) erhaltenen Methacroleins mit Methanol und Sauerstoff in Gegenwart eines heterogenen, Metalle und/oder Metalloxide umfassenden, edelmetallhaltigen Oxidationskatalysators in einem Reaktor und
E) Destillation des aus Schritt D) gewonnenen Produktes in einer Destillationskolonne, wobei ein Methacrolein und Methanol enthaltendes Destillat in den Reaktor gemäß Schritt D) zurückgeführt wird,
wobei es sich bei den Destillationskolonnen gemäß den Schritten C) und E) um dieselbe Destillationskolonne handelt und das Methanol in Schritt C) aus dem Reaktor des Schritts D) stammt und zusammen mit dem aus Schritt D) gewonnenen Produkt in die zweite Destillationskolonne geleitet wird und optional weiteres Methanol in die zweite Destillationskolonne in Schritt E) gegeben wird,
und wobei die Schritte A) bis E) in einem kontinuierlichen Verfahren durchgeführt werden. Dabei ist vor allem Schritt C) neu gegenüber dem Stand der Technik. Erfindungsgemäß ist es gelungen durch eine zusätzliche Destillation zwischen den Schritten B) und D), den Methacrylsäuregehalt in Verfahrensschritt D) überraschend zu reduzieren. Dabei wird die Bildung von Methacrylsäure in Verfahrensschritt D) reduziert. Dadurch ist es weiterhin überraschend gelungen, die Lebensdauer des Katalysators in Schritt D) und damit die Anlagenbelegzeit ohne Stillstand für Wartungsarbeiten, insbesondere für die Katalysatorerneuerung, zu verbessern. Weiterhin konnte überraschend die Ausbeute des Prozesses gegenüber dem Stand der Technik zusätzlich gesteigert und der Verbrauch an Hilfsbase, wie beispielsweise Natriumhydroxid, reduziert werden.
Insbesondere überraschend ist dabei, dass die Gesamtausbeute an MMA insbesondere durch eine Destillation in Gegenwart von Methanol gemäß Schritt C) gesteigert werden kann. Erfindungsgemäß handelt es sich bei den Destillationskolonnen, die in den Schritten C) und E) verwendet werden, um die gleiche Destillationskolonne. Damit ist es sehr überraschend möglich, das neuartige Verfahren mittels nur sehr kleiner Modifikationen, insbesondere mittels einer Modifikation der Leitungsführung, in eine bestehende Anlage zur Herstellung von MMA aus Propanal und Formaldehyd über eine Mannich-Reaktion und eine anschließende direkte oxidative Veresterung mit Methanol zu realisieren. Weiterhin stammt erfindungsgemäß bei dieser Ausführungsform das Methanol in Schritt C) aus dem Reaktor des Schritts D). Dabei wird dieses Methanol zusammen mit dem Produkt aus der oxidativen Veresterung des Schritts D) in die Destillationskolonne des Schritts E) - und damit des Schritts C) - geleitet. Optional kann dabei weiteres Methanol in diese zweite Destillationskolonne gegeben werden, anstelle oder zusätzlich zu der Einleitung des Methanols in den Reaktor des Schritts D). Erfindungsgemäß werden die erfindungsgemäßen Schritte A) bis E) in einem kontinuierlichen Verfahren durchgeführt.
Durch das erfindungsgemäße Verfahren gelingt es auf nicht vorhersehbare Weise, ein Verfahren zur Herstellung von MMA bereitzustellen, das nicht mit den Nachteilen herkömmlicher Verfahren behaftet ist. Insbesondere kann MMA bei einem geringeren Energiebedarf erzeugt werden. Weiterhin kann das Verfahren im Vergleich zum Stand der Technik umweltschonender durchgeführt werden, wobei geringere Mengen an Abfällen erhalten werden und die Atomökonomie wesentlich erhöht wird.

Ein weiterer großer Vorteil des erfindungsgemäßen Verfahrens ist es, dass das bei der Methacroleinsynthese als Nebenprodukt entstehende dimere Methacrolein nicht schon in oder direkt vor Verfahrensschritt B) mit zusätzlichem Aufwand entfernt werden muss, sondern gemäß der bevorzugten Variante des erfindungsgemäßen Verfahrens im ohnehin schon vorhanden Verfahrensschritt C) entfernt wird. Sollte dieses Nebenprodukt nämlich in den Reaktor des Verfahrensschritts D) gelangen, wird es unter Verbrauch von Methanol zu einem entsprechenden Methylester-Nebenprodukt umgesetzt. Somit ist es durch das erfindungsgemäße Verfahren überraschend möglich, zusätzlich die Einsatzmengen an Methanol zu reduzieren. Weiterhin wurde überraschend gefunden, dass das erfindungsgemäß hergestellte MMA und daraus hergestellte Polymere eine geringere Farbzahl aufweisen als Produkte des Standes der Technik, die mittels einer Kombination aus Mannich-Reaktion und oxidativer Veresterung hergestellt wurden.

### Schritte C) und E)

Bei der Destillation des Schritts C) wird die organische Phase aus Schritt B), das Roh-Methacrolein enthaltend, direkt in eine zweite Destillationskolonne, bevorzugt in den mittleren Teil derselben, eingeleitet. Wie bereits ausgeführt handelt es sich bei dieser zweiten Destillationskolonne um die gleiche Destillationskolonne, in die in Schritt E) das Produkt aus Schritt D) überführt wird. In dieser Kolonne erfolgt die Destillation in Gegenwart von Methanol, welches aus Schritt D) zugeleitet und optional durch weitere Zuleitung ergänzt wird.
Bei dieser Destillation werden mindestens zwei Fraktionen erhalten. Das Destillat enthält dabei ein azeotropes Gemisch aus Methanol und Methacrolein. Dieses Destillat wird in den Reaktor gemäß Schritt D) geleitet. Der Sumpf der Kolonne aus Schritt E) enthält überwiegend MMA und Methanol, Wasser, welches noch nach der Destillation in Schritt B) in dem Roh Methacrolein verblieben sein kann, sowie andere höher siedende Bestandteile, wie z.B. dimeres Methacrolein. Außerdem enthält der Sumpf der Kolonne aus Schritt C) bzw. E) Formaldehyd und dessen Folgeprodukte. Bei den Folgeprodukten des Formaldehyds kann es sich insbesondere um Formaldehyd-Hydrat (Methandiol), sowie Oligo- und Polymere von Formaldehyd handeln. In dem erfindungsgemäßen Verfahren, in dem die Schritte C) und E) in der gleichen Kolonne durchgeführt werden, kann optional ein Teilstrom des Roh-Methacroleins aus Schritt B), insbesondere aus dem Phasentrenner, direkt in den Reaktor des Schritts D) bzw. in die Zuleitung des azeotropen Gemischs aus Methacrolein und Methanol von der Kolonne aus Schritt E) in den Reaktor des Schritts D) geleitet werden. Eine solche Ausführungsform ist insbesondere geeignet, in Schritt D) eine höhere Methacrolein-Konzentration zu realisieren. Zwar werden auf diese Weise kleine Mengen Wasser und dimeres Methacrolein zusätzlich in den Reaktor geleitet. Trotzdem wird auch mit einer solchen Ausführungsform die Bildung von Methacrylsäure und der Verbrauch an Methanol reduziert. In der Regel ist dabei das Verhältnis der Methacroleinmenge im Strom aus Schritt B) zur Summe der Methacroleinmengen aus den Schritten B) und E) kleiner als 0,7, bevorzugt kleiner als 0,5, besonders bevorzugt kleiner als 0,3 und ganz besonders bevorzugt kleiner als 0,1.

Bevorzugt wird der Destillationskolonne in Schritt C) bzw. E) noch eine dritte Fraktion entnommen. Bei dieser dritten Fraktion handelt es sich um einen Leichtsiederstrom, der am Kopf der Kolonne C) bzw. E) abgetrennt und entsorgt wird. In Verfahrensschritt E) enthält dieser Leichtsiederstrom beispielsweise Methylformiat. Außerdem kann der Leichtsiederstrom aus der Kolonne C kleine Mengen von Methylal, dem Dimethylacetal von Formaldehyd enthalten. Zur Trennung von der ersten, im vorgehenden Abschnitt als Destillat bezeichneten Fraktion, wird diese erste Fraktion bevorzugt im obersten Drittel der Kolonne als Seitenstrom, nicht jedoch am Kopf der Kolonne entnommen, während der Leichtsiederstrom direkt gasförmig oder kondensiert am Kopf der Kolonne abgeführt und zum Beispiel einer Verbrennung zugeführt wird.

Der Sumpf der Kolonne aus Verfahrensschritt E), der vor allem MMA und Methanol, enthält, wird in einer ersten Alternative in eine Extraktion überführt. In dieser Extraktionsvorrichtung wird der Sumpfstrom unter Zuleitung von Wasser in eine organische und eine wässrige Phase getrennt. Die wässrige Phase wird abgeführt und zum Beispiel zur Verbrennung in einen Thermal Oxidizer oder aber in eine andere Art der Abwasseraufbereitung geleitet.

Ein zusätzlicher Vorteil des erfindungsgemäßen Verfahrens ist weiterhin, dass in Verfahrensschritt C) bzw. E) dimeres Methacrolein, welches nicht vermeidbar in Verfahrensschritt A) gebildet wird, und bei dem es sich um das Produkt einer Diels-Alder-Reaktion zweier Methacrolein-Moleküle handelt, entfernt wird, bevor dieses in den Reaktionsraum der oxidativen Veresterung gemäß Verfahrensschritt D) geleitet wird. In Verfahrensschritt D) würde dieses dimere Methacrolein, wie in den aus dem Stand der Technik bekannten Verfahren, zu einem entsprechenden Methylester umgesetzt. Durch das erfindungsgemäße Verfahren kann unter anderem der Methanolverbrauch des Gesamtprozesses reduziert werden, da dimeres Methacrolein nicht, bzw. in geringerem Umfang in Verfahrensschritt D) verestert wird. Darüber hinaus würde das dimere Methacrolein bei einer begrenzten Katalysatorkapazität in Konkurrenz zur oxidativen Veresterung des monomeren Methacroleins stehen und damit zusätzlich die Raum-Zeit-Ausbeute senken. Dies hat insbesondere eine große Bedeutung, da das dimere Methacrolein gegenüber dem monomeren Methacrolein in der oxidativen Veresterung bevorzugt umgesetzt wird.

Weiterhin würde sich in Verfahrensschritt D) aus dem dimeren Methacrolein auch entsprechende Säure bilden. Diese Säure hat analog zur Methacrylsäure negative Wirkungen auf die Katalysatorstandzeit und müsste daher wie zur Methacrylsäure beschrieben mit einer Hilfsbase neutralisiert werden. Weiterhin verbleibt diese Säure bzw. deren Salz im Kolonnensumpf des Verfahrensschritts E) und kann im späteren Verlauf des Prozesses zu Ablagerung, z.B. in den Destillationskolonnen, führen. Durch das erfindungsgemäße Verfahren wird diese Problematik zusätzlich verhindert.

Bei der in dieser Anmeldung als dimeres Methacrolein oder gleichbedeutend als Dimethacrolein bezeichneten Verbindung handelt es sich um 2,5-Dimethyl-3,4-dihydro-2H-pyran-2-carbaldehyd.
Der erfindungsgemäß nicht oder nur kaum gebildete Methylester des Dimethacroleins stellt in Verfahren des Standes der Technik ein großes Problem dar. Aufgrund gebildeter Azeotrope und einer schlechten Wasserlöslichkeit wird dieser Methylester in die Kolonnen zur MMA-Reinigung geschleppt. Unter den in diesen Kolonnen vorliegenden Bedingungen erfolgt teilweise eine Retro-Diels-Alder Reaktion, bei der neben einem Äquivalent MMA auch ein nun störendes Äquivalent Methacrolein entsteht. Dieses polymerisationsaktive Methacrolein kann dort Nebenreaktionen eingehen, die wiederum zur Bildung von oligomeren Bestandteilen, und einer verminderten Reinheit des Endproduktes führen. Letzteres kann u.a. zu einer erhöhten Farbzahl im Endprodukt führen. Darüber hinaus ist Methacrolein auch in geringen Mengen im Endprodukt in Bezug auf die Toxizität und die allgemeinen Produkteigenschaften problematisch.
Ein weiterer Vorteil der vorliegenden Erfindung ist, dass in Verfahrensschritt C) bzw. E) auch Formaldehydreste aus den Verfahrensschritten A) und B) abgetrennt und damit nicht in Verfahrensschritt D) überführt werden. Die Abtrennung erfolgt im Sumpf der Kolonne als Methandiol und/oder als Oligo- und Polymere des Formaldehyds. Formaldehyd würde in Verfahrensschritt D) gleich zu mehreren Problemen führen.
Formaldehyd selbst wirkt reduktiv, was zu einer Schädigung des in der oxidativen Veresterung eingesetzten Katalysators und damit zu verkürzten Katalysatorstandzeiten führen kann. Weiterhin kann sich unter den oxidativen Bedingungen Ameisensäure bilden, die ebenfalls schädlich für den Katalysator ist. Formaldehyd bildet bekanntlich reversibel eine Reihe von Oligo- und Polymeren (Paraformaldehyd). Die Hydroxyendgruppen solcher Oligo- und Polymeren aus Formaldehyd werden im Laufe der oxidativen Veresterung von Methacrolein umgesetzt, was zu nun stabilen und meistens unlöslichen Polymeren führt. Diese Polymere können wiederum zu Verstopfungen der Apparate und Verringerung der Katalysatorstandzeit führen.

Der Betrieb der Kolonne des Schritts E), in die nur die Reaktionsmischung aus Schritt D), nicht jedoch das Roh-Methacrolein aus Schritt B) eingespeist wird, ist beispielsweise in JP2006-225401 beschrieben. Hier wird auch beschrieben, dass es vorteilhaft ist, die Reaktionsmischung in die Mitte der Kolonne einzuspeisen. Dieser Lehre folgend ist es erfindungsgemäß entsprechend bevorzugt, dass sowohl die Reaktionsmischung des Schrittes D) als auch das Roh-Methacrolein des Schritts B) in die Mitte der Kolonne des Schritts E) geleitet werden. In einer zweiten Alternative wird der Sumpf der Destillationskolonne aus Verfahrensschritt E), unabhängig von der sonstigen Ausführungsform der Erfindung, in einen Phasentrenner überführt. Dort wird der eingeleitete MMA haltige Sumpf in eine organische und eine wässrige Phase getrennt. Diese Alternative bietet sich vor allem bei höherem Wassergehalt des Sumpfes an.
Die aus diesen Alternativen gewonnene organische Phase kann zur weiteren Aufarbeitung des MMA in weitere, ein bis vier in Reihe geschaltete Destillationskolonnen gefahren werden. Dabei kann zum Beispiel abwechselnd die MMA haltige Phase erst als Destillat der jeweiligen Kolonne und darauf als Sumpf der Kolonne gewonnen werden. Auf diese Weise können sowohl höher siedende als auch niedrig siedende Verunreinigungen nahezu vollständig aus dem MMA entfernt werden.
Erfindungsgemäß bevorzugt weist der in den Reaktor gemäß Schritt D) geleitete Strom aus Schritt C) bzw. E) - zuvor als Destillat der zweiten Destillationskolonne bezeichnet - neben Methanol und Methacrolein eine Wassermenge auf, die kleiner ist als die Wassermenge im Roh-Methacrolein aus Schritt B).

Besonders bevorzugt weist der aus Schritt C) bzw. E) nach Schritt D) geleitete Seitenstrom einen Wassergehalt kleiner 0,5 Gew%, besonders bevorzugt kleiner 0,1 Gew% und insbesondere bevorzugt kleiner 500 ppm auf.

In der vorliegenden Erfindung gibt es im Hinblick auf die Bauart der verwendeten Destillationskolonnen keine besondere Einschränkung. Es können beliebige Arten herkömmlicher Destillationskolonnen, Bodenkolonnen oder gepackte Kolonnen, eingesetzt werden.

Da jedoch Roh-Methacrolein, das in den Destillationsturm in Schritt C) bzw. E) eingeleitet wird, eine leicht polymerisierbare Verbindung ist, ist es bevorzugt, eine Destille mit einer Struktur einzusetzen, bei der ein Zusetzen mit Polymerisationsprodukten nicht auftritt und/oder die Polymerisationsprodukte leicht entfernt werden können. Spezifische Beispiele von Destillationstürmen umfassen Bodenkolonnen, die mit einem Siebboden, Kaskadenboden, Turbogridboden, Riffelboden oder dergleichen ausgestattet sind, und gepackte Kolonnen, die mit Packungsmaterialien regelmäßig (wie z.B. Mellapak von Sulzer) oder unregelmäßig (wie z.B. Raschig Superring von Raschig) gepackt sind.

In dem erfindungsgemäßen Verfahren variiert die geeignete Destillationstemperatur im Destillationsturm in Abhängigkeit vom Destillationsdruck, der Zusammensetzung der Flüssigkeit im Destillationsturm, der Anzahl der Böden im Destillationsturm und dergleichen. Um jedoch die Bildung der vorstehend genannten Polymerisationsprodukte und die Bildung von hochsiedenden Verbindungen, die einem Ausbeuteverlust an Methacrolein oder MMA darstellen, auf ein Minimum zu beschränken, ist es bevorzugt, dass die Destillationstemperatur so gering wie möglich ist. Wenn die Destillationstemperatur jedoch zu gering gewählt wird, können Nachteile auftreten. Zu diesen gehören beispielsweise, dass der Destillationsdruck auch gering gewählt werden muss. Hierdurch kann es erforderlich sein, den Destillationsturm in einer unvorteilhaften Größe einzusetzen. Weiterhin kann der Einsatz eines Kühlmittels zum Kondensieren der Gasphase am obersten Tel des Destillationsturmes erforderlich werden. Vorzugsweise liegt die Destillationstemperatur oder die Temperatur der Flüssigkeit in der Kolonne im Bereich von 20 bis 100 °C, besonders bevorzugt von 40 bis 85 °C. Der Destillationsdruck ergibt sich aus dieser Temperatur.

Weiterhin sei darauf hingewiesen, dass die Begriffe Destillationsturm, Destillationskolonne und Kolonne in diesem Text synonym verwendet werden.

### Schritte A) und B)

Das erfindungsgemäße Verfahren umfasst in Verfahrensschritt A) die Herstellung von Methacrolein durch Umsetzung von Propanal mit Formaldehyd über eine Aldol- bzw. Mannichkondensation. Das Formaldehyd kann dabei beispielsweise als Formalin eingesetzt werden. Die hierzu geeigneten Verfahren sind dem Fachmann bekannt und Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2. Insbesondere sei auch auf die besonders bevorzugte Ausführung dieses Schritts A), wie sie in der Europäischen Patentanmeldung mit dem Anmeldeaktenzeichen 13002076.1 beschrieben ist, verwiesen.
Bevorzugt wird die Umsetzung in Verfahrensschritt A) in Gegenwart von 0,1 bis 20 Mol% eines sekundären Amins und 0,1 bis 20 Mol% Säure, jeweils bezogen auf das Propanal, bei einer Temperatur von 100 bis 300 °C und bei einem Druck von 5 bis 100 bar durchgeführt.

Bei der Herstellung von Methacrolein aus Propanal und Formaldehyd wird das Reaktionsgemisch in Schritt B) einer Kolonne zugeleitet und dort bevorzugt mit Wasserdampf gestrippt. Das Methacrolein verlässt zusammen mit Wasser die Kolonne am Kopf. Das Gemisch wird kondensiert und bevorzugt über einen Phasentrenner, insbesondere ein Phasentrenngefäß, in eine obere und eine untere Phase getrennt. Die obere Phase enthält das Methacrolein, Reste Formaldehyd, Propanal und das Nebenprodukt Dimethacrolein und wird in die Destillation gemäß Schritt C) und anschließend zur oxidativen Veresterung gemäß Schritt D) zu MMA weitergeleitet. Die untere Phase besteht hauptsächlich aus Wasser. Vorzugsweise kann sie zur Entfernung des noch darin gelösten Methacroleins zumindest teilweise wieder in die Kolonne zurückgeführt werden.

In der Regel befindet sich zwischen der Destillationskolonne und dem Phasentrenner noch ein Kondensator.

Der Wassergehalt des Roh-Methacroleins aus Schritt B) kann temperaturbedingt variieren. Vorzugsweise wird die nach der Umsetzung von Formaldehyd mit Propanal erhaltene Reaktionsmischung demgemäß auf eine Temperatur abgekühlt, bei der sich der Wassergehalt in der Methacroleinphase einstellt. Vorzugsweise kann die Temperatur im Phasentrenner zwischen 0 und 50 °C, vorzugsweise 5 bis 30 °C und besonders bevorzugt 10 bis 25 °C eingestellt werden.

Die wässrige Katalysatorlösung kann am Sumpf der Kolonne zusammen mit dem bei der Reaktion gebildeten Wasser und dem Wasser aus der eingesetzten Formaldehydlösung abgezogen werden. Für die Weiterverarbeitung kann, wenn sehr wenig Amin und/oder Säure eingesetzt wird und deshalb die Rückführung des Katalysators nicht mehr lohnt, die Sumpfflüssigkeit verworfen werden.

Bei größeren Amin- und/oder Säurekonzentrationen im Sumpfablauf kann aber auch Wasser teilweise destillativ abgetrennt und die verbleibende Katalysatorlösung wieder in den Reaktor zurückgeführt werden. Außerdem ist es möglich, den Sumpfablauf so in zwei Teilströme aufzuteilen, dass ein Teilstrom gerade die Menge an Wasser mit sich führt, die bei der Reaktion gebildet und mit den Ausgangsstoffen eingegeben wurde. Dieser Teilstrom wird dann ausgeschleust und der restliche Anteil in den Reaktor zurückgeführt. Wässriges Formaldehyd und Propanal können auch getrennt vorgeheizt und dem Reaktor zugefahren werden. Auch ist es möglich, das Wasser aus dem Sumpfablauf mittels einer oder mehrerer Membrantrennstufen zu isolieren.

### Schritt D)

Erfindungsgemäß wird das in Schritt A) erhaltene und in den Schritten B) und C) aufgearbeitete Methacrolein in einer direkten oxidativen Veresterungsreaktion in Schritt D) zu MMA umgesetzt. Unter einer direkten oxidativen Veresterungsreaktion wird im Rahmen der vorliegenden Erfindung ein Verfahren verstanden, bei dem Methacrolein in Gegenwart von Methanol und einem Oxidationsmittel, vorzugsweise Sauerstoff, direkt, das heißt ohne die Bildung von großen Mengen an Methacrylsäure, zu MMA umgesetzt wird. Insbesondere sei auch auf die besonders bevorzugte Ausführung dieses Schritts D), wie sie in der Europäischen Patentanmeldung mit dem Anmeldeaktenzeichen 13002076.1 beschrieben ist, verwiesen.

Bei der oxidativen Veresterungsreaktion gemäß Schritt D) werden bevorzugt heterogene Oxidationskatalysatoren, die eines oder mehrere ultrafein verteilte Metalle mit einer durchschnittlichen Teilchengröße von < 20 nm umfassen, eingesetzt. Bei diesen Metallen handelt es sich bevorzugt um Gold, Palladium, Ruthenium, Rhodium oder Silber, bzw. um deren Mischungen. Weiterhin bevorzugt wird die Reaktion in Schritt D) bei einem Druck von 1 bis 100 bar, bevorzugt von 2 bis 50 bar, in flüssiger Phase durchgeführt. Bevorzugt liegt die Temperatur der Reaktion in Schritt D) in einem Bereich von 10 bis 200 °C.

Bevorzugt erfolgt die oxidative Veresterungsreaktion gemäß Schritt D) mit einem molaren Verhältnis von Methanol zu Methacrolein im Bereich von 1:1 bis 50:1.

Erfindungsgemäß entstehen aus Methacrolein bei der oxidativen Veresterungsreaktion gemäß Schritt D) höchstens 2 Gew%, bevorzugt höchstens 1 Gew%, besonders bevorzugt höchstens 0,5 Gew% freier Methacrylsäure, die mit einem basischen Hilfsmittel, wie z.B. Natriumhydroxid. teilweise oder vollständig neutralisiert wird.
Vorzugsweise beträgt der Wassergehalt der in Schritt D) zur oxidativen Veresterung stationär vorliegenden Reaktionsmischung höchstens 5,0 Gew%. Dabei ist der größte Teil dieses Wassers, solches, das bei der oxidativen Veresterung in Schritt D) gebildet wird.
Durch die oxidative Veresterung unter den vorstehend genannten Bedingungen wird ein Reaktionsgemisch, das MMA als Hauptreaktionsprodukt enthält, erhalten. Zusätzlich zu MMA enthält das erhaltene Reaktionsgemisch auch nicht umgesetztes Methacrolein und nicht umgesetztes Methanol und bei der Reaktion gebildetes Wasser und eventuell die genannten Mengen Methacrylsäure als Nebenprodukte. Das Reaktionsgemisch kann außerdem andere Komponenten und Nebenprodukte, die sehr kleine Mengen dimeres Methacrolein, dessen Methylester und andere umfassen, enthalten.
Aufgrund der Polymerisierbarkeit dieser Bestandteile ist es bevorzugt, dass ein oder mehrere Polymerisationsinhibitoren dem Prozess, insbesondere in den Destillationsturm gemäß Verfahrensschritt C) und E) zugegeben werden. Polymerisationsinhibitoren, wie beispielsweise Hydrochinone, Hydrochinonether, wie Hydrochinonmonomethylether oder Di-tert-butylbrenzcatechin, Phenothiazin, N,N'-(Diphenyl)-p-phenylendiamin, 4 Hydroxy-2,2,6,6-tetramethylpiperidin-1-oxyl, p-Phenylendiamin, Methylenblau oder sterisch gehinderte Phenole, sind in der Fachwelt weithin bekannt. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der Polymerisation auftretenden freien Radikale funktionieren. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

### Bezugszeichenliste

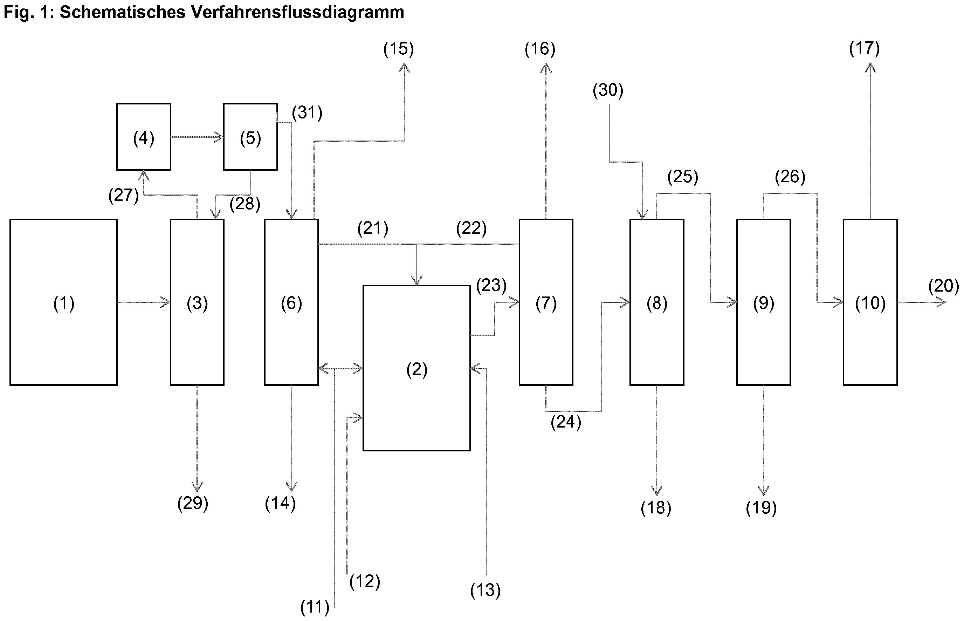

**Fig.1** stellt schematisch die wichtigsten Anlagenbestandteile zur Durchführung eines nicht erfindungsgemäßen Verfahrens in der Ausführungsform mit getrennten Destillationskolonnen für die Schritte C) und E) dar.
- (1): Reaktor für Schritt A)
- (2): Reaktor für Schritt D)
- (3): Destillationskolonne für Schritt B)
- (4): Kondensator der Destillationskolonne (3)
- (5): Phasentrenner aus Schritt B)
- (6): Destillationskolonne aus Schritt C)
- (7): Destillationskolonne aus Schritt E)
- (8): Trennung wässrige und organische Phase (optional)
- (9): Abtrennung hochsiedender Bestandteile (optional)
- (10): Abtrennung niedrigsiedender Bestandteile
- (11): Zuleitung Methanol; wahlweise in Destillationskolonne (6) und/oder in Reaktor (2); optional auch Zuleitung einer Stabilisatorlösung
- (12): Zuleitung Luft bzw. Sauerstoff
- (13): Zuleitung für weitere Prozessstoffe, wie z.B. Hilfsbase oder ähnliches
- (14): Abwasser, optional ganz oder teilweise zur Zurückführung in (1), (3) oder (5)
- (15): Optionales Abgas
- (16): Abgas
- (17): Abgas
- (18): Abwasser
- (19): Hochsiedender Abfall
- (20): Vorgereinigtes MMA zur optionalen weiteren Reinigung, z.B. durch weitere Destillation
- (21): Zuleitung des Destillats aus (6) (Stufe C)), enthaltend ein Gemisch aus Methacrolein und Methanol in (2)
- (22): Zuleitung des Destillats aus (7) (Stufe E)), enthaltend ein Gemisch aus Methacrolein und Methanol in (2)
- (23): Zuleitung des Reaktionsgemischs aus (2) (Stufe D)) in (7) (für Stufe E))
- (24): Leitung des MMA enthaltenden Sumpfes aus Stufe E) (7) in die Trennstufe (8)
- (25): Leitung der organischen Phase aus (8) in Destillationskolonne (9)
- (26): Leitung des Destillats aus (9) in Destillationskolonne (10)
- (27): Leitung des Brüdens aus (3) in Kondensator (4)
- (28): Leitung des Kondensats aus (4) in Phasentrenner (5)
- (29): Abwasser, optional ganz oder teilweise zur Zurückführung in (1)
- (30): Optionale Zuleitung Wasser oder wässrige Säure in (8)
- (31): Zuleitung der organischen Phase des Phasentrenners (5) aus Schritt B) in die Destillationskolonne (6) gemäß Schritt C)

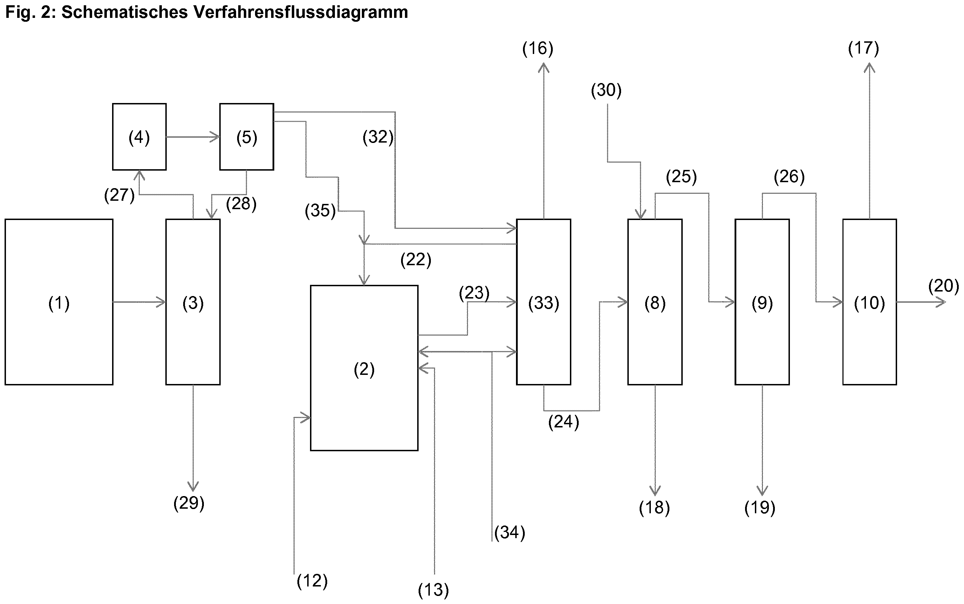

**Fig.2** stellt schematisch die wichtigsten Anlagenbestandteile zur Durchführung des erfindungsgemäßen Verfahrens in der bevorzugten Ausführungsform mit einer gemeinsamen Destillationskolonne für die Schritte C) und E) dar.
- (1): Reaktor für Schritt A)
- (2): Reaktor für Schritt D)
- (3): Destillationskolonne für Schritt B)
- (4): Kondensator der Destillationskolonne (3)
- (5): Phasentrenner aus Schritt B)
- (8): Trennung wässrige und organische Phase (optional)
- (9): Abtrennung hochsiedender Bestandteile (optional)
- (10): Abtrennung niedrigsiedender Bestandteile
- (12): Zuleitung Luft bzw. Sauerstoff
- (13): Zuleitung für weitere Prozessstoffe, wie z.B. Hilfsbase oder ähnliches
- (16): Abgas
- (17): Abgas
- (18): Abwasser
- (19): Hochsiedender Abfall
- (20): Vorgereinigtes MMA zur optionalen weiteren Reinigung, z.B. durch weitere Destillation
- (22): Zuleitung des Destillats aus (33) (Stufe E)), enthaltend ein Gemisch aus Methacrolein und Methanol in (2)
- (23): Zuleitung des Reaktionsgemischs aus (2) (Stufe D) in (33) (für Stufe E))
- (24): Leitung des MMA enthaltenden Sumpfes aus Stufe E) (33) in die Trennstufe (8)
- (25): Leitung der organischen Phase aus (8) in Destillationskolonne (9)
- (26): Leitung des Destillats aus (9) in Destillationskolonne (10)
- (27): Leitung des Brüdens aus (3) in Kondensator (4)
- (28): Leitung des Kondensats aus (4) in Phasentrenner (5)
- (29): Abwasser, optional ganz oder teilweise zur Zurückführung in (1)
- (30): Optionale Zuleitung Wasser oder wässrige Säure in (8)
- (32): Zuleitung der organischen Phase des Phasentrenners (5) aus Schritt B) in die Destillationskolonne (33) gemäß Schritt C)
- (33): Destillationskolonne für die Schritte C) und E)
- (34): Zuleitung Methanol; wahlweise in Destillationskolonne (33) und/oder in Reaktor (2); optional auch Zuleitung einer Stabilisatorlösung
- (35): Optionale Leitung für Teilstrom aus Phasentrenner (5) in Reaktor (2)

Zu den Zeichnungen sei angemerkt, dass weitere dem Fachmann bekannte Komponenten zusätzlich in der Anlage zur Durchführung des erfindungsgemäßen Verfahrens enthalten sein können. So weist beispielsweise in der Regel jede der aufgeführten Kolonnen einen Kondensator auf. In den Zeichnungen ist nur für die erste Kolonne (3) zur Durchführung des Verfahrensschrittes B) ein entsprechender Kondensator (4) aufgeführt.
Auch sei angemerkt, dass in den Zeichnungen nicht jede bevorzugte Ausführungsform berücksichtigt ist. So kann beispielsweise auch die Ausführungsform gemäß Fig.1, mit getrennten Kolonnen (6) und (7) für die Verfahrensschritte C) und E) eine optionale Leitung (35) für den Teilstrom aus Phasentrenner (5) in Reaktor (2) aufweisen, auch wenn diese Leitung (35) nicht in Fig.1 berücksichtigt ist.
Die Position der Zuleitungen gibt nicht deren reale Lage an, sondern weist nur darauf hin in welchen Apparat die Zuleitung geführt wird.

## Patentansprüche

1. Verfahren zur Herstellung von Methylmethacrylat, umfassend die Schritte:
A) Herstellung von Methacroleins aus Propanal und Formaldehyd in Gegenwart von Aminsalzen,
B) Isolierung von flüssigem Roh-Methacrolein durch Destillation in einer ersten Destillationskolonne und anschließende Trennung von einer wässrigen Phase,
C) Destillation des aus Schritt B) gewonnenen Roh-Methacroleins in einer zweiten Destillationskolonne in Gegenwart von Methanol,
D) anschließende oxidative Veresterung des aus Schritt C) erhaltenen Methacroleins mit Methanol und Sauerstoff in Gegenwart eines heterogenen, Metalle und/oder Metalloxide umfassenden, edelmetallhaltigen Oxidationskatalysators in einem Reaktor und
E) Destillation des aus Schritt D) gewonnenen Produktes in einer Destillationskolonne, wobei ein Methacrolein und Methanol enthaltendes Destillat in den Reaktor gemäß Schritt D) zurückgeführt wird,
wobei es sich bei den Destillationskolonnen gemäß den Schritten C) und E) um dieselbe Destillationskolonne handelt und das Methanol in Schritt C) aus dem Reaktor des Schritts D) stammt und zusammen mit dem aus Schritt D) gewonnenen Produkt in die zweite Destillationskolonne geleitet wird und optional weiteres Methanol in die zweite Destillationskolonne in Schritt E) gegeben wird,
und wobei die Schritte A) bis E) in einem kontinuierlichen Verfahren durchgeführt werden.

2. Verfahren gemäß Anspruch 1, **dadurch gekennzeichnet, dass** weiteres Methanol in die zweite Destillationskolonne in Schritt E) gegeben wird.

3. Verfahren gemäß Anspruch 1 oder 2, **dadurch gekennzeichnet, dass** ein Teilstrom des Roh-Methacroleins aus Schritt B) in den Reaktor gemäß Schritt D) geleitet wird, und dass das Verhältnis dieses Teilstroms zum Strom aus der Destillationskolonne gemäß Schritt E) in den Reaktor gemäß Schritt D) zwischen 1 zu 2 und 1 zu 20 liegt.

4. Verfahren gemäß mindestens einem der Ansprüche 1 bis 3, **dadurch gekennzeichnet, dass** in Verfahrensschritt C) bzw E) Dimethacrolein mit dem Kolonnensumpf entfernt wird und eine Dimethacrolein-Menge in Verfahrensschritt D) eingeleitet wird, die kleiner ist als die Menge im Strom, die aus Verfahrensschritt B) in Verfahrensschritt C) eingeleitet wird.

5. Verfahren gemäß mindestens einem der Ansprüche 1 bis 4, **dadurch gekennzeichnet, dass** der zur oxidativen Veresterungsreaktion gemäß Schritt D) eingesetzte heterogene Oxidationskatalysator eines oder mehrere ultrafein verteilte Metalle mit einer durchschnittlichen Teilchengröße von < 20 nm umfasst, welches ausgewählt ist aus der Gruppe bestehend aus Gold, Palladium, Ruthenium, Rhodium und Silber, und dass die Reaktion in Schritt D) bei einem Druck von 1 bis 100 bar in flüssiger Phase durchgeführt wird.

6. Verfahren gemäß mindestens einem der Ansprüche 1 bis 5, **dadurch gekennzeichnet, dass** die Umsetzung gemäß Schritt D) bei einem Druck im Bereich von 2 bis 50 bar und bei einer Temperatur im Bereich von 10 bis 200 °C in flüssiger Phase durchgeführt wird.

7. Verfahren gemäß mindestens einem der Ansprüche 1 bis 6, **dadurch gekennzeichnet, dass** in der Destillationskolonne gemäß Schritt E) ein Methylformiat enthaltender Leichtsiederstrom am Kopf abgetrennt und entsorgt wird.

8. Verfahren gemäß mindestens einem der Ansprüche 1 bis 7, **dadurch gekennzeichnet, dass** die oxidative Veresterungsreaktion gemäß Schritt D) mit einem molaren Verhältnis von Methanol zu Methacrolein im Bereich von 1:1 bis 50:1 erfolgt.

9. Verfahren gemäß mindestens einem der Ansprüche 1 bis 8, **dadurch gekennzeichnet, dass** der Sumpf der zweiten Destillationskolonne gemäß Schritt E) in eine Extraktion überführt wird, in der mit Wasser der Strom in eine organische und eine wässrige Phase getrennt wird.

10. Verfahren gemäß mindestens einem der Ansprüche 1 bis 8, **dadurch gekennzeichnet, dass** der Sumpf der zweiten Destillationskolonne gemäß Schritt E) in einen Phasentrenner überführt wird und dort in eine organische und eine wässrige Phase getrennt wird.

11. Verfahren gemäß Anspruch 9 oder 10, **dadurch gekennzeichnet, dass** die jeweils organische Phase in mindestens einem weiteren Destillationsschritt weiter gereinigt wird.

12. Verfahren gemäß mindestens einem der Ansprüche 1 bis 11, **dadurch gekennzeichnet, dass** der in den Reaktor gemäß Schritt D) geleitete Strom aus Schritt C) bzw. E), umfassend Methanol und Methacrolein, eine Wassermenge aufweist, die kleiner ist als die Wassermenge im Roh-Methacroleins aus Schritt B).

13. Verfahren gemäß mindestens einem der Ansprüche 1 bis 12, **dadurch gekennzeichnet, dass** die Trennung von derwässrigen Phase nach Schritt B) in einem Phasentrenner erfolgt.

14. Verfahren gemäß mindestens einem der Ansprüche 1 bis 13, **dadurch gekennzeichnet, dass** die Umsetzung in Verfahrensschritt A) in Gegenwart von 0,1 bis 20 Mol% sekundären Amin, und 0,1 bis 20 Mol% Säure, jeweils bezogen auf das Propanal, bei einer Temperatur von 100 bis 300 °C und bei einem Druck von 5 bis 100 bar durchgeführt wird.

## Claims

1. Process for preparing methyl methacrylate, comprising the steps of:
A) preparing methacrolein from propanal and formaldehyde in the presence of amine salts,
B) isolating liquid crude methacrolein by distillation in a first distillation column and subsequently separating from an aqueous phase,
C) distilling the crude methacrolein obtained from step B) in a second distillation column in the presence of methanol,
D) subsequently oxidatively esterifying the methacrolein obtained from step C) with methanol and oxygen in the presence of a heterogeneous noble metal-containing oxidation catalyst comprising metals and/or metal oxides in a reactor and
E) distilling the product obtained from step D) in a distillation column, and recycling a distillate comprising methacrolein and methanol into the reactor of step D),
wherein the distillation columns of steps C) and E) are the same distillation column and the methanol in step C) originates from the reactor of step D) and is passed together with the product obtained from step D) into the second distillation column and further methanol is optionally added to the second distillation column in step E),
and wherein steps A) to E) are conducted in a continuous process.

2. Process according to Claim 1, **characterized in that** further methanol is added to the second distillation column in step E).

3. Process according to Claim 1 or 2, **characterized in that** a substream of the crude methacrolein from step B) is passed into the reactor of step D), and **in that** the ratio of this substream to the stream from the distillation column of step E) into the reactor of step D) is between 1:2 and 1:20.

4. Process according to at least one of Claims 1 to 3, **characterized in that**, in process step C) or E), dimethacrolein is removed together with the column bottoms and an amount of dimethacrolein less than the amount in the stream which is introduced from process step B) into process step C) is introduced into process step D).

5. Process according to at least one of Claims 1 to 4, **characterized in that** the heterogeneous oxidation catalyst used for the oxidative esterification reaction in step D) comprises one or more ultrafinely divided metals having an average particle size of < 20 nm which are selected from the group consisting of gold, palladium, ruthenium, rhodium and silver, and **in that** the reaction in step D) is conducted at a pressure of 1 to 100 bar in the liquid phase.

6. Process according to at least one of Claims 1 to 5, **characterized in that** the conversion in step D) is conducted at a pressure in the range from 2 to 50 bar and at a temperature in the range from 10 to 200°C in the liquid phase.

7. Process according to at least one of Claims 1 to 6, **characterized in that**, in the distillation column of step E), a low boiler stream comprising methyl formate is removed overhead and disposed of.

8. Process according to at least one of Claims 1 to 7, **characterized in that** the oxidative esterification reaction in step D) is effected with a molar ratio of methanol to methacrolein in the range from 1:1 to 50:1.

9. Process according to at least one of Claims 1 to 8, **characterized in that** the bottoms from the second distillation column of step E) are transferred into an extraction in which water is used to separate the stream into an organic phase and an aqueous phase.

10. Process according to at least one of Claims 1 to 8, **characterized in that** the bottoms from the second distillation column of step E) are transferred into a phase separator and separated therein into an organic phase and an aqueous phase.

11. Process according to Claim 9 or 10, **characterized in that** the respective organic phase is purified further in at least one further distillation step.

12. Process according to at least one of Claims 1 to 11, **characterized in that** the stream from step C) or E), comprising methanol and methacrolein, passed into the reactor of step D) includes an amount of water less than the amount of water in the crude methacrolein from step B).

13. Process according to at least one of Claims 1 to 12, **characterized in that** the separation from the aqueous phase after step B) is effected in a phase separator.

14. Process according to at least one of Claims 1 to 13, **characterized in that** the conversion in process step A) is conducted in the presence of 0.1 to 20 mol% of secondary amine, and 0.1 to 20 mol% of acid, based in each case on the propanal, at a temperature of 100 to 300°C and at a pressure of 5 to 100 bar.

## Revendications

1. Procédé de fabrication de méthacrylate de méthyle, comprenant les étapes suivantes :
A) la fabrication de méthacroléine à partir de propanal et de formaldéhyde en présence de sels d'amines,
B) l'isolement de méthacroléine brute liquide par distillation dans une première colonne de distillation, puis la séparation d'une phase aqueuse,
C) la distillation de la méthacroléine brute obtenue à l'étape B) dans une deuxième colonne de distillation en présence de méthanol, puis
D) l'estérification oxydative de la méthacroléine obtenue à l'étape C) avec du méthanol et de l'oxygène en présence d'un catalyseur d'oxydation hétérogène comprenant des métaux et/ou des oxydes de métaux, contenant des métaux nobles, dans un réacteur et
E) la distillation du produit obtenu à l'étape D) dans une colonne de distillation, un distillat contenant de la méthacroléine et du méthanol étant recyclé dans le réacteur selon l'étape D),
les colonnes de distillation selon les étapes C) et E) étant la même colonne de distillation et le méthanol à l'étape C) provenant du réacteur de l'étape D) et étant introduit conjointement avec le produit obtenu à l'étape D) dans la deuxième colonne de distillation, et du méthanol supplémentaire étant éventuellement ajouté dans la deuxième colonne de distillation à l'étape E),
et les étapes A) à E) étant réalisées par un procédé continu.

2. Procédé selon la revendication 1, **caractérisé en ce que** du méthanol supplémentaire est ajouté dans la deuxième colonne de distillation à l'étape E).

3. Procédé selon la revendication 1 ou 2, **caractérisé en ce qu'**un courant partiel de la méthacroléine brute de l'étape B) est introduite dans le réacteur selon l'étape D), et **en ce que** le rapport entre ce courant partiel et le courant issu de la colonne de distillation selon l'étape E) dans le réacteur selon l'étape D) est compris entre 1 sur 2 et 1 sur 20.

4. Procédé selon au moins l'une quelconque des revendications 1 à 3, **caractérisé en ce qu'**à l'étape de procédé C) ou E), de la diméthacroléine est éliminée avec le fond de la colonne, et une quantité de diméthacroléine est introduite à l'étape de procédé D), qui est inférieure à la quantité dans le courant qui est introduit depuis l'étape de procédé B) dans l'étape de procédé C).

5. Procédé selon au moins l'une quelconque des revendications 1 à 4, **caractérisé en ce que** le catalyseur d'oxydation hétérogène utilisé pour la réaction d'estérification oxydative selon l'étape D) comprend un ou plusieurs métaux ultra-finement divisés, ayant une taille de particule moyenne < 20 nm, qui sont choisis dans le groupe constitué par l'or, le palladium, le ruthénium, le rhodium et l'argent, et **en ce que** la réaction à l'étape D) est réalisée à une pression de 1 à 100 bar dans la phase liquide.

6. Procédé selon au moins l'une quelconque des revendications 1 à 5, **caractérisé en ce que** la réaction selon l'étape D) est réalisée à une pression dans la plage allant de 2 à 50 bar et à une température dans la plage allant de 10 à 200 °C dans la phase liquide.

7. Procédé selon au moins l'une quelconque des revendications 1 à 6, **caractérisé en ce qu'**un courant de composants de point d'ébullition faible contenant du formiate de méthyle est séparé par la tête dans la colonne de distillation selon l'étape E) et mis au rebut.

8. Procédé selon au moins l'une quelconque des revendications 1 à 7, **caractérisé en ce que** la réaction d'estérification oxydative selon l'étape D) a lieu avec un rapport molaire entre le méthanol et la méthacroléine dans la plage allant de 1:1 à 50:1.

9. Procédé selon au moins l'une quelconque des revendications 1 à 8, **caractérisé en ce que** le fond de la deuxième colonne de distillation est transféré selon l'étape E) dans une extraction, dans laquelle le courant est séparé avec de l'eau en une phase organique et une phase aqueuse.

10. Procédé selon au moins l'une quelconque des revendications 1 à 8, **caractérisé en ce que** le fond de la deuxième colonne de distillation est transféré selon l'étape E) dans un séparateur de phases, et y est séparé en une phase organique et une phase aqueuse.

11. Procédé selon la revendication 9 ou 10, **caractérisé en ce que** la phase organique respective est davantage purifiée dans au moins une étape de distillation supplémentaire.

12. Procédé selon au moins l'une quelconque des revendications 1 à 11, **caractérisé en ce que** le courant introduit dans le réacteur selon l'étape D) issu de l'étape C) ou E), comprenant du méthanol et de la méthacroléine, comprend une quantité d'eau qui est inférieure à la quantité d'eau dans la méthacroléine brute issue de l'étape B).

13. Procédé selon au moins l'une quelconque des revendications 1 à 12, **caractérisé en ce que** la séparation de la phase aqueuse selon l'étape B) a lieu dans un séparateur de phases.

14. Procédé selon au moins l'une quelconque des revendications 1 à 13, **caractérisé en ce que** la réaction à l'étape de procédé A) est réalisée en présence de 0,1 à 20 % en moles d'une amine secondaire et de 0,1 à 20 % en moles d'un acide, à chaque fois par rapport au propanal, à une température de 100 à 300 °C et à une pression de 5 à 100 bar.
